# EUROPEAN PATENT APPLICATION

(11) **EP 2 374 428 A1**
(43) Date of publication of application: **12.10.2011**
(21) Application number: 10159674.0
(22) Date of filing: 12.04.2010
(51) Int. Cl.: A61C 3/03, B22F 3/22, A61B 17/32

(54) **Dental and medical ultrasonic tip and method of manufacturing the same**

(71) Applicant: CetaTech, Inc. Technological Innovation Center for Mechanical parts of Automobiles, Yonghyeon-myeon Sacheon-si, Gyeongsangnam-do 664-953 (KR); B&L Biotech Co., Ltd., 1136-1 Sanbon-dong Gunpo-si, Gyeonggi-do 435-040 (KR)
(72) Inventor: Kwon, Young San, 135-970, Seoul (KR); Lee, In Hwan, 135-110, Seoul (KR); Baek, Seongho, 135-110, Seoul (KR)
(74) Representative: Zimmermann, Tankred Klaus

(57) **Abstract**

Provided are a dental and medical ultrasonic tip that may enhance a durability and a reliability and may simplify a manufacturing process, and a method of manufacturing the same. The method of manufacturing the dental and medical ultrasonic tip may include: providing a molding mixture including powder; manufacturing an intermediate molded object by injecting the molding mixture so as to integrally form a tip body and a plurality of tip protrusions protruded from the tip body; and sintering the intermediate molded object. The tip body and the plurality of tip protrusions may be formed using the same material, and the plurality of tip protrusions may be closely arranged on a substantially polished portion of the tip body.

## Description

### BACKGROUND

### 1. Field of the Invention

The present invention relates to a dental and medical ultrasonic tip and a method of manufacturing the same, and more particularly, to a dental and medical ultrasonic tip that may enhance a durability and a reliability, and simplify a manufacturing process, and a method of manufacturing the same.

### 2. Description of the Related Art

A dental ultrasonic tip is used to remove tartar attached onto teeth and other attached substances by vibrating a tip using the vibration energy of ultrasonic waves, or is used for an endodontic treatment, for example, a root cap operation. In addition, the dental ultrasonic tip is widely used for various types of dental treatments such as dental implant surgery, periodontal surgery, oral surgery, maxillofacial surgery, and the like. The dental ultrasonic tip may be used minimizing damage to soft tissues, for example, nerves, blood vessels, muscles, and the like, when performing all types of treatments, such as osteotomy and drilling that are an orthopedic treatment with respect to a bone, saucerization of thinly removing a cortical bone, an oscillation by a mechanical saw blade that is a beauty-oriented procedure, rasp of a bone using a reciprocation and a polishing tool, and the like.

In general, the dental ultrasonic tip may have a sharp distal end having a decreasing cross-sectional area while approaching the distal end. A coating layer having particles, for example, diamond particles may be coated on the tip to form protrusions. A contact and a friction between the dental ultrasonic tip and an operation portion may be made when diamond particles of the coating layer protruded from the tip contact with the operation portion.

The conventional dental ultrasonic tip may be manufactured by a general mechanical processing such as a cutting, a polishing, and the like. Therefore, it is difficult to perform precise processing and an error rate may increase. Also, since an additional processing process needs to be added to form the coating layer on the conventional dental ultrasonic tip, a number of manufacturing processes and costs may increase.

As described above, the conventional dental ultrasonic tip may be formed by the mechanical processing and thus may not enhance a processing precision to be greater than a predetermined level and may use only a material suitable for the mechanical processing. In addition, manufacturing costs may increase and a processing speed may decrease, which may act as disadvantages for a mass production.

In the conventional dental ultrasonic tip, the coating layer formed on the tip may be partially come off due to repetitive contacts and abrasions with teeth. Accordingly, a treatment efficiency and a medical stability may decrease. In particular, particles, for example, diamond particles of the conventional dental ultrasonic tip may be easily come off from the coating layer. Accordingly, a durability and a lifespan may be deteriorated and a precision and a convenience of treatment may be deteriorated.

Accordingly, there is a need for a dental ultrasonic tip that may enhance a durability and a reliability and may also simplify a manufacturing process.

### SUMMARY

An aspect of the present invention provides a dental and medical ultrasonic tip that may simplify a manufacturing process and decrease manufacturing costs, and a method of manufacturing the same.

Another aspect of the present invention also provides a dental and medical ultrasonic tip that may be advantageous for a mass production due to a quick and easy manufacturing process, may enhance a processing precision, and may use a material without some constraints on the material, and a method of manufacturing the same.

Another aspect of the present invention also provides a dental and medical ultrasonic tip that may enhance a durability and a reliability and may also expand a lifespan, and a method of manufacturing the same.

Another aspect of the present invention also provides a dental and medical ultrasonic tip that may enhance a treatment efficiency and may also enhance a precision and a convenience of treatment, and a method of manufacturing the same.

According to an aspect of the present invention, there is provided a method of manufacturing a dental and medical ultrasonic tip, including: providing a molding mixture containing powder; manufacturing an intermediate molded object by injecting the molding mixture so as to integrally form a tip body and a plurality of tip protrusions protruded from the tip body; and sintering the intermediate molded object. The tip body and the plurality of tip protrusions may be formed using the same material, and the plurality of tip protrusions may be closely arranged on a substantially polished portion of the tip body.

In this instance, a metal power may be used for the powder and a non-metal power may be mixed with the metal power and thereby be used for the power. A functional additive may be mixed to enhance various types of physical characteristics.

The shape, the size, and the arraignment of each of the tip protrusions may variously vary depending on requirements and design specifications. For example, each of the tip protrusions may be provided on a non-contact portion to be in a contactable shape according to at least one of a point contact, a line contact, and a sideface contact. Each of the tip protrusions may be provided in at least one of a circular conic shape, a polygonal conic shape (e..g, a quadrangular polygonal conic shape, a triangular polygonal conic shape, and the like), a frustum shape of the circular conic shape and the polygonal conic shape (e.g., a truncated circular conic shape, a truncated polygonal conic shape, and the like), a circular cylindrical shape, a polyhedral shape (e.g., a pentahedral shape, a hexahedral shape, and the like), and an ellipsoidal solid shape. Also, each of the tip protrusions may be provided in at least one of a shape such as a triangular screw having a triangular vertical cross-section and being continuously protruded along a spiral direction or a circumferential direction and a shape such as a quadrangular screw having a quadrangular vertical cross-section and being continuously protruded along the spiral direction or the circumferential direction.

The tip body may be formed in a linear shape to have a predetermined length, and may also be formed in a curved shape. The tip body may be formed to have a distal end having a decreasing cross-sectional area while approaching the distal end. Depending on requirements, the tip body may be provided to have a circular cross-section or a polygonal cross-section such as a triangle, a quadrangle, and the like.

Also, the tip body may be formed to have the overall same thickness and width. In this case, each of the tip protrusions may be formed to have a decreasing size while approaching the distal end of the tip body. The entire shape of the dental and medical ultrasonic tip may be provided in a taper shape due to the plurality of tip protrusions each having a different size. Also, all the tip protrusions may be formed to have the same size and shape.

The molding mixture may include a binder. Prior to the sintering of the intermediate molded object, debinding the intermediate molded object may be added to remove at least one portion of the binder. For example, the binder may be removed through a solvent debinding process using a solvent and a thermal debinding process using a heat.

The dental and medical ultrasonic tip may vibrate according to the vibration energy of ultrasonic waves, and may be used to remove tartar attached onto teeth and other attached substances and may also be used for various types of endodontic treatments, for example, a root cap operation. Also, the dental and medical ultrasonic tip may be widely used for various types of dental treatments such as dental implant surgery, periodontal surgery, oral surgery, maxillofacial surgery, and the like. In addition to the dental treatments, the dental and medical ultrasonic tip may be used minimizing damage to soft tissues, for example, nerves, blood vessels, muscles, and the like, when performing all types of treatments, such as osteotomy and drilling that are an orthopedic treatment with respect to a bone, saucerization of thinly removing a cortical bone, an oscillation by a mechanical saw blade that is a beauty-oriented procedure, rasp of a bone using a reciprocation and a polishing tool, and the like.

### EFFECT

According to embodiments of the present invention, there may be provided a dental and medical ultrasonic tip and a method of manufacturing the same. In this instance, the dental and medical ultrasonic tip having a plurality of integrated tip protrusions may be manufactured through a powder injection process without using an additional process. Accordingly, it is possible to simplify a manufacturing process and to decrease manufacturing costs.

Also, according to embodiments of the present invention, since a manufacturing process of a dental and medical ultrasonic tip may be simplified, it may be advantageous for a mass production. Compared to a mechanical processing, it is possible to significantly enhance a processing precision. Also, when a material unsuitable for the mechanical processing is used, it is possible to easily manufacture the dental and medical ultrasonic tip.

Also, according to embodiments of the present invention, a tip body and a plurality of tip protrusions may be provided not as separate parts but an integrated single part. Therefore, it is possible to prevent or decrease separation or alienation of the tip protrusions occurring due to a contact between a dental and medical ultrasonic tip and teeth.

Also, according to embodiments of the present invention, it is possible to extend a lifespan of a product and to enhance a precision and a convenience of a treatment.

Also, according to embodiments of the present invention, there is no particular constraint on a size of a tip protrusion, a shape thereof, a number of tip protrusions, and an arrangement thereof. Accordingly, it is possible to readily manufacture a dental and medical ultrasonic tip having various tip protrusions depending on requirements and design specifications. Also, depending on requirements, the tip protrusions may be provided in a regular arrangement.

Also, according to embodiments of the present invention, when an intermediate molded object is sintered, pores may be removed in the intermediate molded object. When the intermediate molded object is shrunk, tip protrusions constituting the intermediate molded object may also be shrunk. Accordingly, it is possible to form the tip protrusions having a minimum size.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a flowchart illustrating a method of manufacturing a dental and medical ultrasonic tip according to an embodiment of the present invention;
FIG. 2 is a process diagram to describe a method of manufacturing a dental and medical ultrasonic tip according to an embodiment of the present invention;
FIG. 3 is a perspective view illustrating a structure of a dental and medical ultrasonic tip according to an embodiment of the present invention;
FIG. 4 is a side view illustrating a structure of a dental and medical ultrasonic tip according to an embodiment of the present invention;
FIG. 5 is a cross-sectional view cut along a line I-I of FIG. 4; and
FIGS. 6 to 11 are perspective views illustrating a structure of a dental and medical ultrasonic tip according to other embodiments of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

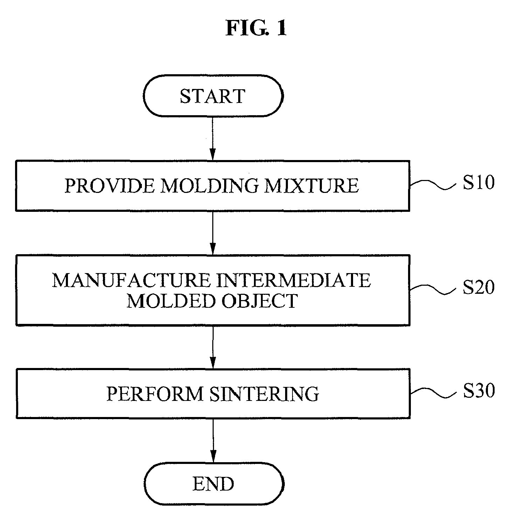

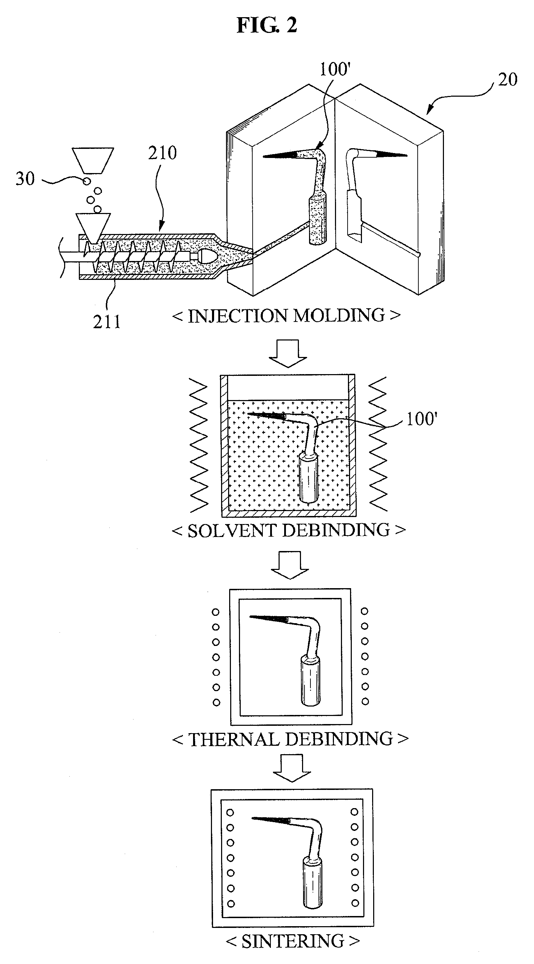

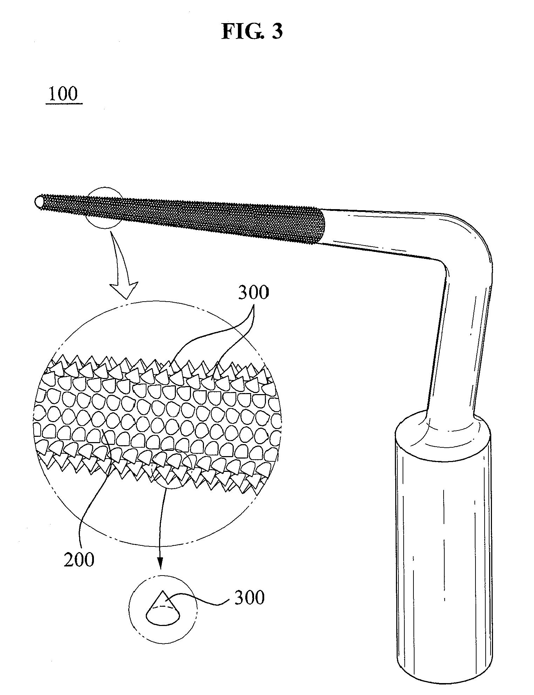

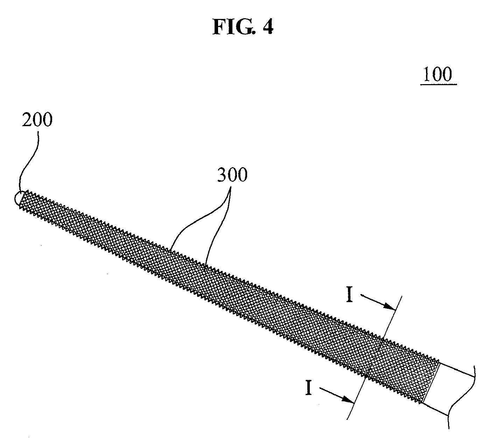

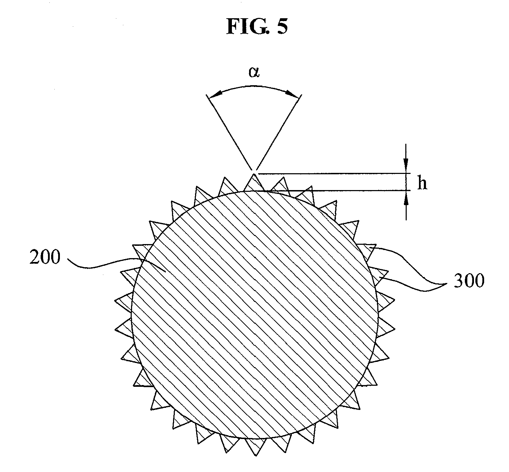

FIG. 1 is a flowchart illustrating a method of manufacturing a dental and medical ultrasonic tip 100 according to an embodiment of the present invention, and FIG. 2 is a process diagram to describe a method of manufacturing the dental and medical ultrasonic tip 100 according to an embodiment of the present invention. FIG. 3 is a perspective view illustrating a structure of the dental and medical ultrasonic tip 100 according to an embodiment of the present invention, FIG. 4 is a side view illustrating a structure of the dental and medical ultrasonic tip 100 according to an embodiment of the present invention, and FIG. 5 is a cross-sectional view cut along a line I-I of FIG. 4.

As illustrated in figures, the dental and medical ultrasonic tip 100 according to an embodiment of the present invention may be manufactured through a predetermined manufacturing process as follows:

The dental and medical ultrasonic tip 100 may be manufactured through operation S10 of providing a molding mixture 30 including powder, operation S20 of manufacturing an intermediate molded object 100' including a tip body 200 and a plurality of tip protrusions 300 by injecting the molding mixture 30, the tip body 200 and the plurality of tip protrusions 300 being integrally formed on the tip body 200 and the plurality of tip protrusions 300 being protruded from a portion of the tip body 200 substantially polished by a contact with teeth, and operation S30 of sintering the intermediate molded object 100'.

Initially, the molding mixture 30 including the powder may be provided. The molding mixture 30 may be formed on a feedstock unit of a predetermined size to enable the molding mixture 30 to be readily supplied to a general injection molding unit 210.

General metal powder or a mixture thereof may be used as the powder. Depending on embodiments, in addition to the metal powder, general non-metal powder and a mixture thereof may be used. Also, a functional additive may be added to enhance various types of physical characteristics and a bio-suitability. For example, the power may use the metal power such as a stainless steel base (e.g., austenite such as 316 and 304), a martensite base such as 420 and 440, precipitation hardening such as 630 and 631, a ware-resistance (e.g., SKD, SCM, and the like), a titanium alloy (e.g., Ti, Ti-6Al-4V, and the like), or a mixture thereof. Depending on embodiments, in addition to the metal powder, the non-metal powder such as hard metal (WC-Co), ceramic (zirconia, alumina, and the like), and a mixture of the non-metal powder may be used for the powder.

The molding mixture 30 may be provided together with the binder and thus the powder and the binder may be mixed at a predetermined mixture ratio in an appropriate temperature and thereby be provided. For example, in operation S10 of providing the molding mixture 30, compared to a total volume of a base material including the powder, the binder of about 30% to about 70% may be mixed with the base material whereby the injection moldable molding mixture 30 may be provided. Depending on embodiments, the ratio of the binder to the powder may variously vary and thus the present invention is not limited thereto or restricted thereby.

The binder may be mixed so as to assign a fluidity enabling the powder that is a raw material to be uniformly injected into a metallic pattern 20 during an injection process, and to enhance a strength of the intermediate molded object 100' molded from the metallic pattern 20. A general single binder may be used for the binder and various types of binders having different melting points may be used for the binder. For example, paraffin wax, polyethylene, polypropylene, stearic acid, and the like may be mixed with each other at a fixed amount to thereby constitute the binder. In addition, a bonding agent, a lubricant, a plasticizer, a surfactant, and the like, and a mixture thereof may be added as the binder. In operation S 10, the powder and the binder may be uniformly mixed using a general twin screw extrusion type mixer, a twin blade mixer, and the like.

Next, the intermediate molded object 100' including the tip body 200 and the plurality of tip protrusions 300 being integrally formed with the tip body 200 and being protruded from the tip body 200 may be molded using the molding mixture 30 through a powder injection molding (PIM) scheme.

Specifically, the molding mixture 30 may be placed into a cylinder 211 of the injection molding unit 210, be transported while being plasticized, and be injected from a nozzle of the cylinder 211 into the metallic pattern 20 and then be cooled and solidified. Through the above process, the intermediate molded object 100' in a shape of the dental and medical ultrasonic tip 100 may be obtained.

In operation S20 of manufacturing the intermediate molded object frame 100', a shape, a size, and an arrangement of the tip protrusion 300 may variously vary depending on requirements and design specifications. For example, the tip protrusion 300 may be provided on a non-contact portion to be in a contactable shape according to one of a point contact, a line contact, and a sideface contact. The tip protrusion 300 may be provided in one of a circular conic shape, a polygonal conic shape (e..g, a quadrangular polygonal conic shape, a triangular polygonal conic shape, and the like), a frustum shape of the circular conic shape and the polygonal conic shape (e.g., a truncated circular conic shape, a truncated polygonal conic shape, and the like), a circular cylindrical shape, a polyhedral shape (e.g., a pentahedral shape, a hexahedral shape, and the like), and an ellipsoidal solid shape. Also, the tip protrusion 300 may be provided in one of a shape such as a triangular screw having a triangular vertical cross-section and being continuously protruded along a spiral direction or a circumferential direction, and a shape such as a quadrangular screw having a quadrangular vertical cross-section and being continuously protruded along the spiral direction or the circumferential direction (see FIG. 3, and FIG. 6 to FIG. 10).

In operation S20 of manufacturing the intermediate molded object 100', the tip body 200 may be formed in a linear shape to have a predetermined length, and may also be formed in a curved shape. The tip body 200 may be formed in a general taper shape to a decreasing cross-sectional area while approaching a distal end. Depending on requirements, the tip body 200 may be provided to have a circular cross-section or a polygonal cross-section such as a triangle, a quadrangle, and the like.

Also, the tip body 200 may be formed to have the overall same thickness and width. In this case, the tip protrusion 300 may be formed to have a decreasing size as the tip protrusion 300 comes closer to the distal end of the tip body 200 from a proximal end of the tip body 200. The entire shape of the dental and medical ultrasonic tip 100 may be provided in a taper shape due to the plurality of tip protrusions 300 each having a different size. Also, all the tip protrusions 300 may be formed to have the same size and shape.

Next, a debinding process using a general debinding equipment (not shown) may be performed to remove a portion of the binder contained in the intermediate molded object 100'.

When the binder is configured to include a plurality of binders having different characteristics, for example, different melting points, the binder may be removed by performing debinding several times. For example, the binder may be removed by a solvent debinding using a solvent and a thermal debinding using a heat.

Specifically, the solvent debinding of the intermediate molded object 100' to remove a wax such as a paraffin wax in the binder may be performed according to a general solvent debinding scheme using a solvent such as an N-hexane, a heptanes, a thinner, and the like. The thermal debinding by heating the solvent debound intermediate molded object to remove a remaining binder may be performed according to a general thermal debinding scheme. Depending on embodiments, instead of the debinding scheme applied to the aforementioned debinding process, a general debinding scheme such as an electrolytic degreasing, an ultrasonic degreasing, and the like may be applicable.

In the case of the metal powder, the thermal debinding may include performing a preliminary sintering of excluding an oxidation of the intermediate molded object 100', which may occur during the thermal debinding, by heating the intermediate molded object 100' within a heating furnace maintaining a reductive or inactive atmosphere, and of enhancing a strength of the intermediate molded object 100' by increasing the temperature of a thermal debinding furnace after the thermal debinding.

The manufacturing process of the dental and medical ultrasonic tip 100 may be completed by performing sintering in a general sintering furnace (not shown). Here, the general sintering furnace may maintain a predetermined temperature condition and atmosphere in order to remove pores of the intermediate molded object 100', and to maintain a mechanical strength.

A sintering temperature required during the above process may be slightly different depending on a type of powder that is a material of the intermediate molded object 100', a particle size, a purity, and a type of additive. However, in the case of stainless powder, the sintering may be performed in a state where a sintering furnace temperature condition is maintained at around about 1000 °C to about 1500 °C. A sintering time may vary depending on required physical properties, however, about 1 minute to about 5 hours may be appropriate.

Also, the thermal debinding for removing the binder and the sintering of sintering the intermediate molded object 100' in which the binder is removed may be simultaneously performed using the sintering furnace. In this case, the thermal debinding may be performed together while sintering the intermediate molded object in which the binder is partially removed through the solvent debinding. Accordingly, a number of processes may be reduced, which may result in bringing a good product productivity.

In addition, it is very difficult to process a tip protrusion of tens of microns using a general mechanical processing. However, according to an embodiment of the present invention, when the intermediate molded object 100' is sintered, pores may be removed within the intermediate molded object 100' and the intermediate molded object 100' may be shrunk. Also, since the intermediate molded object 100' is shrunk, the tip protrusion 300 may also be shrunk. Accordingly, the tip protrusion 300 may be formed to have a minimum size. Also, since the density of the intermediate molded object 100' formed by injection molding is uniform, sinter shrinkages in directions X, Y, and Z may be the same. Accordingly, each of the tip protrusions 300 may also maintain a uniform size.

The dental and medical ultrasonic tip 100 manufactured by the above manufacturing method may vibrate due to ultrasonic frequency, be used to remove tartar attached onto teeth and other attached substances, and may also be used for various types of endodontic treatments, for example, a root cap operation. Also, the dental and medical ultrasonic tip 100 may be widely used for various types of dental treatments such as dental implant surgery, periodontal surgery, oral surgery, maxillofacial surgery, and the like. In addition to the dental treatments, the dental and medical ultrasonic tip 100 may be used minimizing damage to soft tissues, for example, nerves, blood vessels, muscles, and the like, when performing all types of treatments, such as osteotomy and drilling that are an orthopedic treatment with respect to a bone, saucerization of thinly removing a cortical bone, an oscillation by a mechanical saw blade that is a beauty-oriented procedure, rasp of a bone using a reciprocation and a polishing tool, and the like.

Hereinafter, the dental and medical ultrasonic tip 100 including the tip body 200 and the plurality of tip protrusions 300 being integrally formed with the tip body 200 and being protruded from the tip body 200 is described with reference to FIGS. 3 to 5 by taking an example of forming the tip protrusions 300 in a circular conic shape to be contactable on a non-contact portion.

As described above, the tip body 200 and the plurality of tip protrusions 300 are provided not as separate parts but as a single integrally formed part.

The tip body 200 may be formed in a linear shape to have a circular cross-section and to have a predetermined length. In the present embodiment, the tip body 200 is formed to have the circular cross-section and to have a decreasing diameter while approaching a distal end of the tip body 200. Depending on embodiments, a plurality of taper faces (not shown) may be formed on the tip body 200 for the tip body 200 to have a decreasing polygonal cross-section while approaching the distal end of the tip body 200. For example, the surface of the tip body 200 may be divided into four taper surfaces, and may also be divided into two taper surfaces, three taper surfaces, or at least five taper surfaces.

Each of the tip protrusions 300 is formed in a circular conic shape having a sharp vertex and a circular base side, and the plurality of tip protrusions 300 is integrally formed with the tip body 200 on the whole surface of the tip body 200. An axis of the tip protrusion 300 may be vertically provided with respect to the base side. Depending on embodiments, the axis of the tip protrusion 300 may be provided to be inclined at a predetermined angle with respect to the base side. An inclined angle α of the tip protrusion 300 and a height h of the tip protrusion 300 may variously vary depending on requirements and treatment environments.

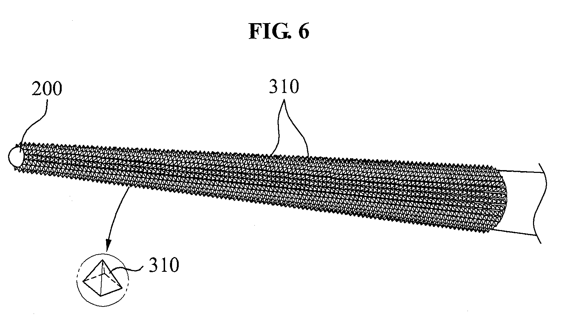

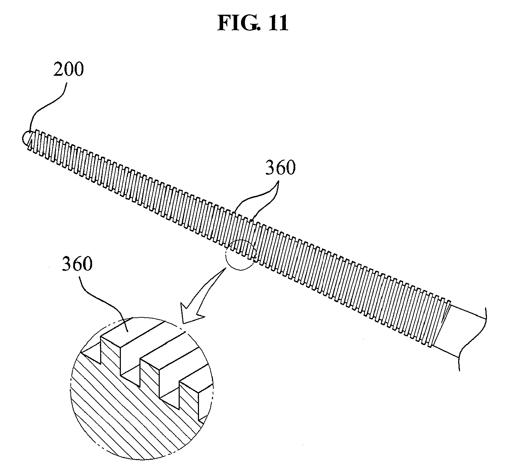

Hereinafter, a dental and medical ultrasonic tip according to other embodiments of the present invention will be described. FIGS. 6 to 11 are perspective views illustrating a structure of a dental and medical ultrasonic tip according to other embodiments of the present invention. Like reference numerals are assigned to the same or equivalent elements and thus further descriptions related thereto will be omitted here.

As shown in FIG. 6, a dental and medical ultrasonic tip according to another embodiment of the present invention may include a tip body 200 and a plurality of tip protrusions 310 protruded from the tip body 200 to be integrally formed with the tip body 200. In this instance, the tip body 200 may be formed to have a decreasing cross-sectional area, for example, a circular cross-section or a polygonal cross-section while approaching a distal end. The tip protrusion 310 may be formed in a quadrangular conic shape having a sharp vertex and a quadrangular base side, and be protruded from the tip body 200 to be integrally formed with the tip body 200.

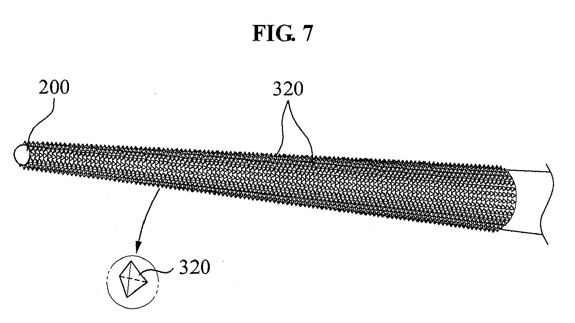

As shown in FIG. 7, a dental and medical ultrasonic tip according to still another embodiment of the present invention may include a tip body 200 and a plurality of tip protrusions 320 protruded from the tip body 200 to be integrally formed with the tip body 200. In this instance, the tip body 200 may be formed to have a decreasing cross-sectional area, for example, a circular cross-section or a polygonal cross-section while approaching a distal end. The tip protrusion 320 may be formed in a triangular conic shape having a sharp vertex and a triangular base side, and be protruded from the tip body 200 to be integrally formed with the tip body 200.

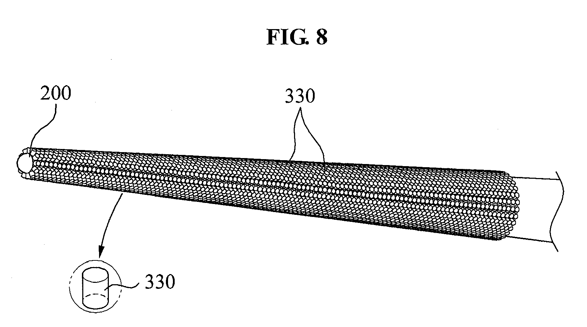

As shown in FIG. 8, a dental and medical ultrasonic tip according to yet another embodiment of the present invention may include a tip body 200 and a plurality of tip protrusions 330 protruded from the tip body 200 to be integrally formed with the tip body 200. In this instance, the tip body 200 may be formed to have a decreasing cross-sectional area, for example, a circular cross-section or a polygonal cross-section while approaching a distal end. The tip protrusion 330 may be formed in a frustum shape having a circular or polygonal cross-section as a cutting edge, and be protruded from the tip body 200 to be integrally formed with the tip body 200. Here, the frustum may include a truncated circular cone such as an oblique cylinder that is a frustum of a general circular cone, a truncated quadrangular cone such as an oblique parallelepiped that is a frustum of a general quadrangular cone, and a truncated triangular cone such as an oblique parallel pentahedron that is a frustum of a general triangular cone, and may also include a polyhedron such as a hexahedron, a pentahedron, and the like. A shape and a structure of the frustum may variously vary depending on requirements and design specifications. Therefore, the present invention is not limited thereto or restricted thereby.

In the aforementioned embodiment, the contact surface, that is, a top surface of the tip protrusion 330 formed in the frustum shape may have the same size and shape as the base side. Depending on embodiments, the contact surface of the tip protrusion 330 may have a size or a shape different from the base side such as a general truncated circular cone or a truncated pyramid.

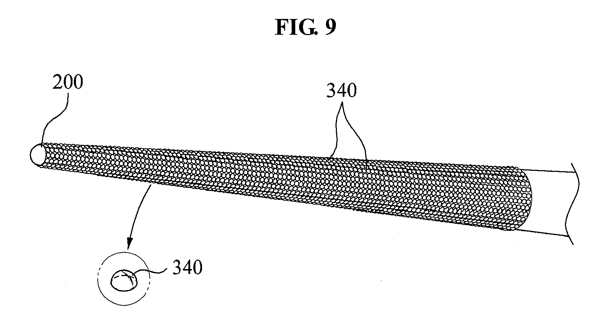

As shown in FIG. 9, a dental and medical ultrasonic tip according to a further another embodiment of the present invention may include a tip body 200 and a plurality of tip protrusions 340 protruded from the tip body 200 to be integrally formed with the tip body 200. In this instance, the tip body 200 may be formed to have a decreasing cross-sectional area, for example, a circular cross-section or a polygonal cross-section while approaching a distal end. The tip protrusion 340 may be formed in a hemispherical or ellipsoidal solid shape, and be protruded from the tip body 200 to be integrally formed with the tip body 200.

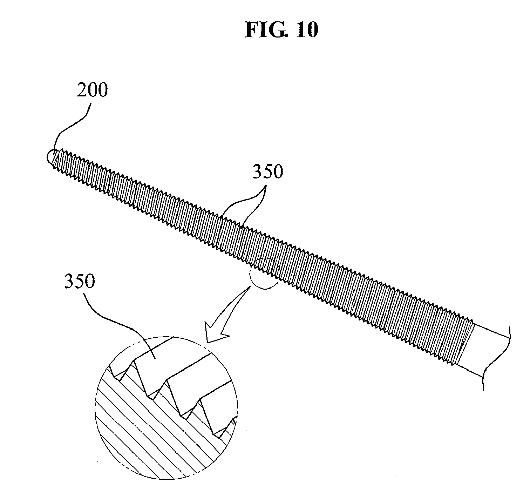

As shown in FIG. 10, a dental and medical ultrasonic tip according to still another embodiment of the present invention may include a tip body 200 and a plurality of tip protrusions 350 protruded from the tip body 200 to be integrally formed with the tip body 200. In this instance, the tip body 200 may be formed to have a decreasing cross-sectional area, for example, a circular cross-section or a polygonal cross-section while approaching a distal end. The tip protrusion 350 may be formed in a shape such as a triangular screw having a triangular vertical cross-section and being continuously protruded along a spiral direction or a circumferential direction, and be protruded from the tip body 200 to be integrally formed with the tip body 200.

As shown in FIG. 1, a dental and medical ultrasonic tip according to still another embodiment of the present invention may include a tip body 200 and a plurality of tip protrusions 360 protruded from the tip body 200 to be integrally formed with the tip body 200. In this instance, the tip body 200 may be formed to have a decreasing cross-sectional area, for example, a circular cross-section or a polygonal cross-section while approaching a distal end. The tip protrusion 360 may be formed in a shape such as a quadrangular screw having a quadrangular vertical cross-section and being continuously protruded along the spiral direction or the circumferential direction, and be protruded from the tip body 200 to be integrally formed with the tip body 200.

In each of the aforementioned and illustrated embodiments of the present invention, description is made using a single type of a tip protrusion. However, it is only an example and thus, depending on embodiments, the aforementioned tip protrusions formed to have different shapes may be simultaneously applicable.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. A method of manufacturing a dental and medical ultrasonic tip, comprising:
providing a molding mixture including powder;
manufacturing an intermediate molded object by injecting the molding mixture so as to integrally form a tip body and a plurality of tip protrusions protruded from the tip body; and
sintering the intermediate molded object,
wherein the tip body and the plurality of tip protrusions are formed using the same material, and the plurality of tip protrusions are closely arranged on a substantially polished portion of the tip body.

2. The method of claim 1, wherein each of the tip protrusions is provided on a non-contact portion to be in a contactable shape according to one of a point contact, a line contact, and a sideface contact.

3. The method of claim 2, wherein each of the tip protrusions is provided in one of a circular conic shape, a polygonal conic shape, a truncated circular conic shape, a truncated polygonal conic shape, a circular cylindrical shape, a polyhedral shape, and an ellipsoidal solid shape.

4. The method of claim 2, wherein each of the tip protrusions is provided in one of a shape having a triangular vertical cross-section and being continuously protruded along a spiral direction or a circumferential direction, and a shape having a quadrangular vertical cross-section and being continuously protruded along the spiral direction or the circumferential direction.

5. The method of claim 1, wherein each of the tip protrusions is provided to have the same size or a different size.

6. The method of claim 1, wherein the tip body is provided to have a circular cross-section or a polygonal cross-section.

7. The method of claim 1, wherein:
the molding mixture comprises a binder, and
the method, prior to the sintering, further comprises debinding the intermediate molded object to remove a portion of the binder.

8. A dental and medical ultrasonic tip, comprising:
a tip body; and
a plurality of tip protrusions being integrally formed with the tip body, being protruded from a polished portion of the tip body, and being closely arranged on the tip body,
wherein the tip body and the plurality of protrusions are formed by integrally injecting a molding mixture into a shape corresponding to the tip body and the plurality of tip protrusions, and by simultaneously sintering the molding mixture injected into the shape.

9. The dental and medical ultrasonic tip of claim 8, wherein each of the tip protrusions is provided on a non-contact portion to be in a contactable shape according to one of a point contact, a line contact, and a sideface contact.

10. The dental and medical ultrasonic tip of claim 9, wherein each of the tip protrusions is provided in one of a circular conic shape, a polygonal conic shape, a truncated circular conic shape, a truncated polygonal conic shape, a circular cylindrical shape, a polyhedral shape, and an ellipsoidal solid shape.

11. The dental and medical ultrasonic tip of claim 9, wherein each of the tip protrusions is provided in one of a shape having a triangular vertical cross-section and being continuously protruded along a spiral direction or a circumferential direction, and a shape having a quadrangular vertical cross-section and being continuously protruded along the spiral direction or the circumferential direction.

12. The dental and medical ultrasonic tip of claim 8, wherein each of the tip protrusions is provided to have the same size or a different size.

13. The dental and medical ultrasonic tip of claim 8, wherein the tip body is provided to have a circular cross-section or a polygonal cross-section.
